# EUROPÄISCHE PATENTSCHRIFT

(11) **EP 0 925 110 B1**
(45) Veröffentlichungstag und Bekanntmachung des Hinweises auf die Patenterteilung: **20.03.2002**
(21) Anmeldenummer: 97927004.8
(22) Anmeldetag: 10.06.1997
(51) Int. Cl.: B01J 20/32, C07K 1/14

(54) **KONJUGAT, GEEIGNET ZUR BINDUNG VON SUBSTANZEN**
CONJUGATE USEFUL FOR BINDING SUBSTANCES
CONJUGUE UTILE POUR LIER DES SUBSTANCES

(30) Priorität: 10.06.1996 DE 19623131
(43) Veröffentlichungstag der Anmeldung: 30.06.1999
(73) Patentinhaber: Scholz, G.H. Dr., 04425 Plösitz/Taucha (DE); Huse, K, Dr, 04416 Markkleeberg (DE); Leistner, S, Dr., 04349 Leipzig (DE); Herrmann, Konrad, Dr., 04229 Leipzig (DE); Vieweg, Silke, Dr, 04109 Leipzig (DE)
(72) Erfinder: Scholz, G.H. Dr., 04425 Plösitz/Taucha (DE); Huse, K, Dr, 04416 Markkleeberg (DE); Leistner, S, Dr., 04349 Leipzig (DE); Herrmann, Konrad, Dr., 04229 Leipzig (DE); Vieweg, Silke, Dr, 04109 Leipzig (DE)
(74) Vertreter: Schüssler, Andrea, Dr.
(86) Internationale Anmeldenummer: DE9701208
(87) Internationale Veröffentlichungsnummer: WO9747383

(56) Entgegenhaltungen:
- EP-A- 0 056 977
- WO-A-89/08257
- WO-A-92/16292
- WO-A-95/33557
- US-A- 5 502 022

## Beschreibung

Die Erfindung betrifft Konjugate, die sich zur Bindung von Substanzen eignen, Verfahren zur Herstellung solcher Konjugate sowie deren Verwendung.

Es ist bekannt, Proteine mit Hilfe von T-Gelen zu trennen(vgl. EP-A2-0 165 912, EP-A2-0 168 363). T-Gele sind Adsorbentien, in denen ein Thiol, z.B. Mercaptoethanol, über einen von Divinylsulfon abgeleiteten Linker an einen Träger, z.B. Agarose, gebunden ist. Die Adsorption von Proteinen an T-Gele erfolgt bei hoher Salzkonzentration, z.B. 0,5 M K₂SO₄. Dies ist in zweierlei Hinsicht nachteilig. Zum einen muß die Lösung der zu trennenden Proteine auf die hohe Salzkonzentration eingestellt werden, was zeit- und kostenaufwendig ist. Zum anderen müssen die Lösungen der getrennten Proteine wieder von der hohen Salzkonzentration befreit werden, was vielfach zu einer Beeinträchtigung der Proteinaktivität führt.

Der vorliegenden Erfindung liegt somit die Aufgabe zugrunde, ein Mittel bereitzustellen, mit dem Proteine ohne vorstehende Nachteile getrennt werden können.

Erfindungsgemäß wird dies durch die Gegenstände in den Patentansprüchen erreicht.

Gegenstand der vorliegenden Erfindung ist somit ein Konjugat, umfassend einen Liganden und einen Träger, wobei der Ligand die allgemeine Struktur (1) aufweist

X-S- (1),

in der
- X: ein Chinazolin oder Derivat davon ist, und
- S: ein Schwefelatom ist.

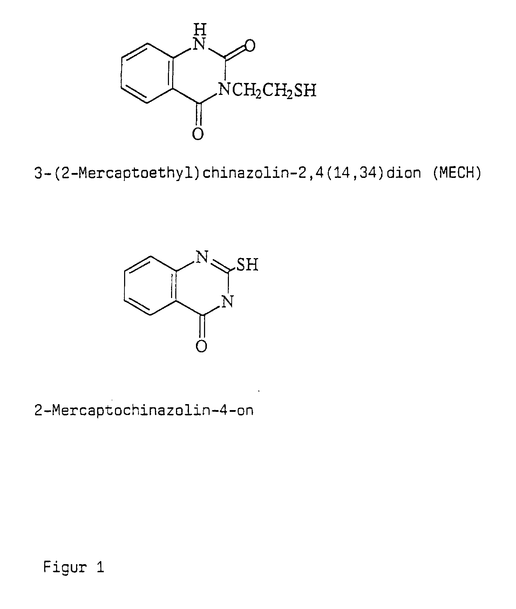

Der Ausdruck "Chinazolin oder Derivat davon" umfaßt Verbindungen jeglicher Art, die die Chinazolin-Struktur aufweisen 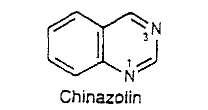 oder davon abgeleitet sind. Vertreter der Chinazolin-Derivate sind Chinazoline, die mit einem aliphatischen, aromatischen und/oder heterozyklischen Rest substituiert sind. Diese Reste können sich an jeder Position des Chinazolins befinden, insbesondere am C₆-Ring. Auch können mehrere dieser Reste vorliegen. Diese können gleich oder verschieden voneinander sein. Vertreter der Chinazolin-Derivate sind z.B. Chinazolinon, wie 4-Chinazolinon, und Chinazolindion, wie 2,4-Chinazolindion (vgl. Fig. 1). Diese können ebenfalls mit vorstehenden Resten substituiert sein. Das Chinazolin oder Derivat davon kann über jede Position des Chinazolin-Rings gebunden sein. Günstigerweise ist das Chinazolin oder Derivat davon über Position 2 oder 3 des Chinazolin-Rings gebunden.

In einem erfindungsgemäßen Konjugat vermittelt S die Bindung zum Träger. Dies kann durch eine direkte Bindung zwischen S und dem Träger oder über einen Linker, der zwischen S und dem Träger vorliegt, erfolgen. Der Ausdruck "Linker" umfaßt Verbindungen jeglicher Art, die zur Verknüpfung von S mit dem Träger fähig sind. Vorzugsweise weist eine solche Verbindung die Struktur -CH₂-CH₂-SO₂-CH₂-CH₂- auf.

In bevorzugter Ausführungsform weist ein erfindungsgemäßes Konjugat zwischen X und S einen Spacer auf. Der Ausdruck "Spacer" umfaßt Verbindungen jeglicher Art, die zur Verknüpfung von X mit S fähig sind. Dies sind z.B. aliphatische Reste, wie -CH₂-CH₂-.

In einem erfindungsgemäßen Konjugat können mehrere Liganden an einen Träger gebunden sein. Diese können gleich oder verschieden voneinander sein. Auch können mehrere Träger, die gleich oder verschieden voneinander sein können, an einen Liganden gebunden sein.

Der Ausdruck "Träger" umfaßt Verbindungen jeglicher Art, an die vorstehende Liganden gebunden sein können. Beispiele für einen Träger sind eine Chromatographiematrix, wie Agarose, Zellulose, Latex und Sepharose, eine Mikrotiterplatte, eine Membran, eine Hohlfaser, Glas, ein magnetisches Teilchen, wie Manetbeads, ein lösliches oder unlösliches Makromolekül, wie Dextran, ein Lipid, eine positive oder negative Ladung-tragende chemische Gruppe, wie Carboxyl-, Sulfonat- und Aminogruppe, ein Photolabel, Biotin, Avidin, ein Peptid, ein Protein, wie ein Antigen, ein Immunglobulin und ein Enzym, ein Arzneistoff, ein strahlendichtes Partikel, wie Goldsole, und eine Nukleinsäure, wie DNA- und RNA-Moleküle.

Erfindungsgemäße Konjugate zeichnen sich dadurch aus, daß sie die verschiedensten Substanzen binden können. Beispiele solcher Substanzen sind Peptide, Proteine, wie Plasmaproteine, Rezeptoren und Immunglobuline, und Peptid(Protein)-Strukturen aufweisende Substanzen, wie Viren, Mikroorganismen und Zellen. Die Substanzen können in den verschiedensten Flüssigkeiten vorliegen. Beispiele solcher Flüssigkeiten sind Körperflüssigkeiten, wie Blut, Lymphe, Urin, Sputum und Liquor, und Überstände bzw. Lysate von Zellen und Geweben. Die Bindung der Substanzen an die erfindungsgemäßen Konjugate kann selektiv sein. Die hierzu notwendigen Bedingungen kann der Fachmann durch übliche Verfahren bestimmen. Beispielsweise wird er zur Bindung von Immunglobulinen eine relativ niedrige Salzkonzentration, z.B. 25mM, und einen pH im Bereich von 5 bis 9 wählen. Die Bindungskapazität erfindungsgemäßer Konjugate ist hoch. Sie liegt z.B. bei >30 mg Substanz (z.B. Protein)/ml Konjugat (z.B. Agarose als Träger). Ferner ist die Bindung zwischen den Substanzen und den erfindungsgemäßen Konjugaten stark und stabil, z.B. gegenüber hohen Temperaturen. Dies kann genutzt werden, weitere Substanzen, z.B. Antigene an die gebundenen Substanzen, z.B. Immungiobuline, zu binden. Gebundene Substanzen können durch übliche Verfahren, wie Behandlung mit Alkalien, z.B. 10mM NaOH und/-oder 10 mM Na₂CO₃, desorbiert werden. Sie liegen dann in einer Form vor, die sich nicht meßbar von jener unterscheidet, welche die Substanzen vor ihrer Bindung an die erfindungsgemäßen Konjugate innehatten.

Erfindungsgemäße Konjugate eignen sich daher zur Anreicherung von verschiedensten Substanzen. Dies können insbesondere Proteine, wie Plasmaproteine, Rezeptoren und Immunglobuline sein. Die Proteine können in den verschiedensten Flüssigkeiten, wie Körperflüssigkeiten und Überständen bzw. Lysaten von Zellen, vorliegen. Desweiteren eignen sich erfindungsgemäße Konjugate zur Entfernung von Substanzen. Dies können insbesondere Peptid(Protein)-Strukturen aufweisende Substanzen, wie Viren, Mikroogranismen und Zellen, sein. Solche Substanzen können in den vorstehenden Flüssigkeiten vorliegen. Die vorliegende Erfindung stellt somit einen wichtigen Beitrag zur kontaminationsfreien Bereitstellung von Substanzen, besonders Proteinen, ganz besonders Immunglobulinen, dar. Des weiteren zeichnet sie sich dadurch aus, daß erfindungsgemäße Konjugate auch kostengünstig eingesetzt werden können. Dies zeigt sich insbesondere gegenüber der Verwendung von T-Gelen bzw. Protein A oder Protein G.

Darüber hinaus eignen sich erfindungsgemäße Konjugate als universelle Bausteine verwendet zu werden, wo Substanzen an einen Träger gebunden werden sollen. Beispielsweise können damit Enzym(Träger)-Antikörper (Substanz)-Konstrukte hergestellt werden. Solche Konstrukte können in diagnostischen Verfahren, wie Immunblot- und Immunhistochemie-Techniken, verwendet werden. Der Antikörper erkennt das nachzuweisende Antigen und bindet daran, während das Enzym diese Bindung durch Umsetzung eines zugegebenen Substrats wiedergibt. Auch können Arzneistoff(Träger)- Antikörper(Substanz)-Konstrukte hergestellt werden. Solche Konstrukte können therapeutisch eingesetzt werden. Der Antikörper erkennt ein zellspezifisches Antigen und bindet daran, während der Arzneistoff, der z.B. ein Zellgift ist, in die Zelle eindringt und diese zerstört.

Die vorliegende Erfindung stellt somit auch einen wichtigen Beitrag zur Bereitstellung von synthetischen Mitteln zur Diagnose und Therapie dar.

Ein weiterer Gegenstand der vorliegenden Erfindung ist ein Verfahren zur Herstellung vorstehender Konjugate. In einem solchen Verfahren kann z.B. von einer Verbindung -X-SH oder -X-Y-SH (vgl. Fig. 1) ausgegangen werden, wobei Y ein Spacer ist. Eine solche Verbindung kann mit einem Träger, z.B. Agarose, kovalent verbunden werden. Der Träger kann eine Linker-Ausgangsverbindung, d.h. eine Verbindung, aus der der Linker gebildet wird, wie Divinylsulfon, enthalten. Alternativ kann auch eine vorstehende Verbindung zunächst mit einer Linker-Ausgangsverbindung und dann mit einem Träger umgesetzt werden. Hierzu kann es günstig sein, die Verbindung zuerst an ein inertes Adsorbens zu immobilisieren und dann nach Umsetzung mit der Linker-Ausgangsverbindung und dem Träger wieder vom Adsorbens zu entfernen.

Kurze Beschreibung der Zeichnungen:
- Fig.: 1: zeigt Verbindungen, die zur Herstellung erfindungsgemäßer Konjugate geeignet sind, und
- Fig.: 2: zeigt ein Elutionsprofil einer Chromatographie von Humanserum an einem erfindungsgemäßen Konjugat, hergestellt aus Sepharose 4B, Divinylsulfon und MECH. Angegeben sind die Extinktion bei 280 nm als Maß für den Proteingehalt ( - ), der Gehalt an Immunglobulin ( . ), der Eulutions-pH-Wert ( o ) und die durchgeführten Pufferwechsel (Pfeile).

Die folgenden Beispiele erläutern die Erfindung:

### Beispiel 1: Reinigung von Fibroblasten-spezifischen, monoklonalen Antikörpern (mAKAS01, mAKAS02) mit einem erfindungsgemäßen Konjugat.

(a) Divinylsulfon-aktivierte Agarose wurde in einem Büchner-Trichter mit 10 Volumen deionisiertem Wasser gewaschen. Anschließend wurde das Gel mit 2 Vol. 0.1 molarer NaOH Lösung äquilibiert. 2g des im Wasserstrahl-Vakuum trocken gesaugten Gels wurden in 5 ml MECH Lösung (30 mg MECH pro 5 ml 0.1 M NaOH) suspendiert und 4 Stunden bei Raumtemperatur unter Rotieren ineinem Gefäß belassen. Danach wurde der Suspension 1 ml Glycerol zugegeben und für weitere 12 Stunden inkubiert. Anschließend wurde das Gel in einem Büchner Trichter mit deionisiertem Wasser gewaschen. Es wurde ein erfindungsgemäßes Konjugat erhalten.
   Zur Einschätzung der Effizienz der Kopplung wurde folgendes Verfahren entwickelt:
   Das mit Wasser gewaschene und im Wasserstrahlvakuum trocken gesaugte Gel wurde in einem definierten Volumen Wasser suspendiert und mit Glycerin in einem Verhältnis von 1:10 gemischt. Von der erhaltenen Suspension wurde ein Absorptionsspektrum in einem Bereich von 230-400 nm angefertigt (Referenzküvette: 90 % Glycerol). In diesem Spektrum lassen sich bei erfolgreicher Kopplung, die für Chinazoline typischen Absorptionen bei ca. 320 nm quantifizieren. Das Ergebnis zeigte, daß die Kopplung quantitiv erfolgte.
   Das Konjugat wurde in eine Chromatographiesäule (Ø 8 mm) gefüllt und mit 25 mM Sörensenpuffer pH 7.4 äquilibriert. Anschließend wurden 50 ml Hybridoma-Zellkulturüberstand (mAKAS 01/02-Konzentration ca. 10 µg/ml) auf die Säule aufgetragen (Flußgeschwindigkeit: 1 ml/min) und mit vorstehendem Sörensenpuffer proteinfrei (O.D. 280 nm < 0.05) gespült. Anschließend wurden die gebundenen Proteine mit 5 ml 10 mM NaOH Lösung eluiert. Die abgelösten mAK wurden in einer Sörensen-Puffervorlage gesammelt.
   Von aufgetragenen 0.5 mg monoklonalen Antikörpern (mAK) wurden 0.45 mg absorbiert und 0.40 mg euliert. Der Nachweis erfolgte mittels Durchflußzytometrie und indirekter Immunfluoreszenz. Die abgelösten (mAK) zeigten hinsichtlich ihrer Bindungsaffinitäten, der Temperatur- und pH-Wert-Stabilität und ihrer Stabilität in verdünnten wässrigen Lösungen keine Unterschiede zu den nichtbehandelten Kulturüberständen.
(b) Die Reinigung von (a) wurde dahingehend verändert, daß zur Elution nicht 10 mM NaOH, sondern 10 mM Na₂CO₃ Lösung verwendet wurde. Von aufgetragenen 0.5 mg mAK wurden 0.45 mg absorbiert und 0.44 mg eluiert. Der Nachweis erfolgte mittels Durchflußzytometrie und indirekter Immunfluoreszenz.
(c) Die Reinigung von (a) wurde dahingehend verändert, daß zur Elution nicht 10 mM NaOH, sondern 10 mM Na₂CO₃ in physiologischer Kochsalzlösung verwendet wurde.
   Von aufgetragenen 0.5 mg mAK wurden 0.45 mg absorbiert und 0.42 eluiert. Der Nachweis erfolgte mittels Durchflußzytometrie und indirekter Immunfluoreszenz.
(d) Die Reinigung von (a) wurde dahingehend verändert, daß das Gel nicht mit 25 mM Sörensenpuffer pH 7.4, sondern mit Phosphat buffered Saline (PBS) pH 7.2 äquilibriert wurde.
   Von aufgetragenen 0.5 mg mAK wurden 0.43 mg absorbiert und 0.40 mg eluiert. Der Nachweis erfolgte mittels Durchflußzytometrie und indirekter Immunfluoreszenz.

### Beispiel 2: Reinigung von Immunglobulinen aus humanem Serum mit einem erfindungsgemäßen Konjugat.

Sepharose 4B wurde in einem Büchner Trichter mit ca. 10 Vol. deionisiertem Wasser gewaschen. Anschließend wurde das Gel mit 2 Vol. 0.5 M Na₂CO₃-Lösung äquilibriert. 2 g des im Wasserstrahlvakuum trocken gesaugten Gels, wurden in 5 ml 0.5 M Na₂CO₃ Lösung suspendiert und mit 0.25 ml Divinylsulfon versetzt. Diese Suspension wurde über Nacht bei Raumtemperatur unter Rotation inkubiert. Das auf diese Weise erhaltene Gel wurde in einem Büchner Trichter mit 10 Vol. eiskaltem deionisiertem Wasser gewaschen. Anschließend wurde das Gel wie in Beispiel 1 mit MECH zur Reaktion gebracht. Es wurde ein erfindungsgemäßes Konjugat erhalten.

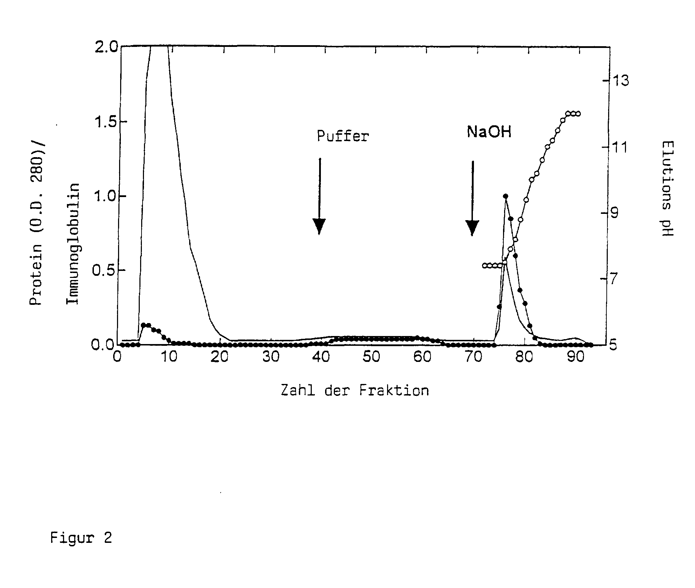

Das Konjugat wurde in eine Chromatographiesäule Ø 8 mm gefüllt und mit 25 mM Sörensenpuffer pH 7.4 äquilibriert. Anschließend wurden 1.0 ml Humanserum (Pool von 200 gesunden Spendern) auf die Säule aufgetragen (Flußgeschwindigkeit: 1 ml/min) und mit vorstehendem Söresenpuffer proteinfrei (O.D. 280 nm < 0.05) gespült. Anschließend wurden die gebundenen Proteine mit 5 ml 10 mM NaOH Lösung eluiert. Das abgelöste Protein wurde in einer Sörensen-Puffervorlage gesammelt. Das Elutionsporfil ist in Fig. 2 gezeigt.

Von aufgetragenen 65 mg Gesamtprotein wurden 14.5 mg absorbiert und 14.0 mg eluiert. Der Immunglobulinnachweis mittels eines kommerziellen ELISA-Tests ergab, daß sich 95 % des aufgetragenen IgG an die Matrix gebunden hatten und 86 % wieder eluiert wurden. Die Bindung traf für alle Subklassen (IgG1-lgG4) zu.

### Beispiel 3: Isolierung von IgG aus humanem Plasma mit einem erfindungsgemäßen Konjugat

Ein Test wurde wie in Beispiel 2 durchgeführt, mit der Ausnahme, daß statt 1 ml Serum, 1 ml humanes Plasma eingesetzt wurde.
Bindung und Elution von Gesamtproteinen und von IgG waren identisch mit den mit Serum erzielten Ergebnissen.

### Beispiel 4: Isolierung von Immunglobulinen aus Kaninchen- bzw. Ziegenserum mit einem erfindungsgemäßen Konjugat

Das Beispiel wurde wie in Beispiel 2 durchgeführt, mit der Ausnahme, daß anstelle von humanem Serum Kaninchenserum bzw. Serum von der Ziege zum Einsatz kam. Mehr als 90 % des aufgetragenen Kaninchen- bzw. Ziegen-IgG banden sich an das Adsorbens und mehr als 85 % wurden eluiert. Die Tatsache, daß sich mehr als 90 % des im Serum befindlichen Immunglobulins gebunden hat, zeigte, daß sich auch alle Ig-Subklassen der untersuchten Spezies an das Konjugat banden.

### Beispiel 5: Isolierung von Immunglobulinen aus humanem Serum mittels Affinitätselution unter Verwendung eines erfindungsgemäßen Konjugats

Der Test wurde wie im Beispiel 2 durchgeführt, mit der Ausnahme, daß zur Elution gebundener Proteine nicht NaOH sondern ein wasserlösliches Ligandenderivat eingesetzt wurde. Dieses Derivat wurde wie folgt hergestellt:
100 mg Mercaptoethylsulfonsäure (MES) wurden mit 10 ml Anionenaustauscherharz (Dowex 1x8, OH-Form) in 10 mM NaOH 30 min. vermischt und das Harz wurde anschließend mit 2 Vol. 10 mM NaOH gewaschen. Das Harz wurde dann in 10 ml 10 mM NaOH resuspendiert und der Suspension wurden 200 µl Divinylsulfon zugesetzt. Der Ansatz wurde 2 Stunden bei Raumtemperatur gerührt und anschließend das Harz auf einem Büchner Trichter mit 20 ml 10 mM NaOH frei von Divinylsulfon gewaschen. Das Harz wurde dann in 10 ml 10 mM NaOH resuspendiert und der Suspension wurden 100 mg MECH zugegeben. Der Ansatz wurde 1 Stunde bei Raumtemperatur gerührt und anschließend das Harz in eine Chromatographiesäule verbracht. Das Harz wurde mit 2 Vol. 10 mM NaOH gewaschen und anschließend mit 50 mM Sörensenpuffer, pH 7.4 gespült. Anschließend wurde mit gleichem Sörensenpuffer, der 1 M NaCI enthielt, die Säule nachgewaschen. Das Eluat wurde fraktioniert und die Fraktionen mit einer O.D. 420 nm > 0.1, vereinigt. Die gepoolten Fraktionen wurden mit 1 N HCI angesäuert und mit Ether extrahiert. Der Etherextrakt wurde zur Trockne gebracht und in 3 ml Sörensenpuffer aufgenommen. Diese Lösung wurde anstelle von NaOH zur Elution eingesetzt.im Eluat ließ sich ca. 75 % des aufgetragenen IgG nachweisen.

### Beispiel 6: Reinigung von Immunglobulinen mit einem bekannten T-Gel (Vergleichsversuch)

Die Herstellung eines T-Gels wurde wie in Beispiel 2 durchgeführt, mit der Ausnahme, daß statt MECH Mercaptoethanol, Mercaptopurin, Mercaptopyrimidin bzw. Mercaptopyridin in der Kopplungsreaktion eingesetzt wurden. Die Chromatographiebedingungen entsprachen denen des Beispiels 2.
Von den aufgetragenen 65 mg Protein wurden weniger als 0.2 mg unter den angegebenen Bedingungen gebunden und eluiert. Mehr als 90 % des IgG fand sich in der nichtgebundenen Fraktion (Säulendurchlauf). Dies zeigt, daß T-Gele Immunglobuline bei niedriger Salzkonzentration nicht ausreichend binden können.

### Beispiel 7: Abtrennung von freier Meerettich-Peroxidase von anti-Kaninchen-IgG (Ziege)-Peroxidase-Verbindung mit einem erfindungsgemäßen Konjugat

Ein Gemisch aus Meerettich-Peroxidase und einer anti-Kaninchen-lgG (Ziege)-Peroxidase-Verbindung wurde auf 1 ml Konjugat von Beispiel 1 aufgetragen und wie vorstehend angegeben chromatographiert. Als Ergebnis zeigte sich, daß die anti-Kaninchen-lgG-Peroxidase-Verbindung gebunden wurde, während die Peroxidase nicht gebunden wurde. Antigenbindung und Enzymaktivität der eluierten Verbindung war unverändert. Somit eignen sich erfindungsgemäße Konjugate, Enzym-Antikörper-Verbindungen von freien Enzymen abzutrennen.

### Beispiel 8: Kopplung eines Liganden an Magnetpartikel (Dynabeads)

Tosyl-aktivierte magnetische Partikel (Dynabeads) wurden mit Dithiothreitol umgesetzt und nachfolgend wurde die verbliebene freie SH-Gruppe des Dithiothreitols mit Divinylsulfon in üblicher Weise umgesetzt. Das so erhaltene Konjugat behielt seine selektiven Bindungseigenschaften. Zur Demonstration der Zeilerkennung wurde ein monoklonaler Antikörper (mAK) gegen CD69 an das Konjugat gebunden. Das mit diesem mAK beladene Konjugat bildete mit PAS aktivierten Lymphozyten Rosetten, die die erhaltene Antigenbindungsfähigkeit der so immobilisierten CD69-mAK belegen. Das vorstehende Konjugat kann somit zur Zelltrennung bzw. zur Negativselektion von Zellverunreinigungen verwendet werden. Vorteilhaft an vorstehendem Konjugat ist, daß mit einem einmal gewonnenen Konjugat nacheinander verschiedene Antikörper immobilisiert werden können, da die Antikörper nicht wie üblich kovalent an den Microbeads gebunden sind.

**Tabelle 1:**

| Bestimmung der Proteinbindungskapazität von 0,5 g erfindungsgemäßem Konjugat, hergestellt aus Agarose, Divinylsulfon und MECH (vgl. Fig. 1) (Als Kontrolle diente Agarose) | |
|---|---|
| aufgetragenes Serum ml (mg Protein) | Eluiertes Protein mg |
| Affinitätsgel | |
| 0.4 (23.8) | 5.77 |
| 0.8 (47.5) | 12.27 |
| 1.0 (59.4) | 17.28 ± 1.2^{a} |
| 1.2 (71.3) | 17.02 |
| 1.6 (95.0) | 16.97 |
| 2.0 (118.8) | 18.89 |

| Kontrollgel | |
|---|---|
| 1.0 (59.4) | 0.5 ± 0.2^{b} |

| | |
|---|---|
| ^{a} Mittelwert und Standardabweichung (n=8) | |
| ^{b} Mittelwert und Standardabweichung (n=5) | |

## Patentansprüche

1. Konjugat, umfassend einen Liganden und einen Träger, wobei der Ligand die allgemeine Struktur (1) aufweist
X-S- (1)
in der
X ein Chinazolin oder Derivat davon ist, und
S ein Schwefelatom ist.

2. Konjugat nach Anspruch 1, **dadurch gekennzeichnet, daß** das Chinazolin-Derivat ein Chinazolinon oder Chinazolindion ist.

3. Konjugat nach Anspruch 2, **dadurch gekennzeichnet, daß** das Chinazolinon ein 4-Chinazolinon ist.

4. Konjugat nach Anspruch 2, **dadurch gekennzeichnet, daß** das Chinazolindion ein 2,4-Chinazolindion ist.

5. Konjugat nach einem der Ansprüche 1 bis 4, **dadurch gekennzeichnet, daß** X mit einem aliphatischen, aromatischen und/oder heterozyklischen Rest substituiert ist.

6. Konjugat nach einem der Ansprüche 1 bis 5, **dadurch gekennzeichnet, daß** das Chinazolin oder Derivat davon über Position 2 oder 3 des Chinazolin-Rings gebunden ist.

7. Konjugat nach einem der Ansprüche 1 bis 6, **dadurch gekennzeichnet, daß** zwischen S und dem Träger ein Linker vorliegt.

8. Konjugat nach Anspruch 7, **dadurch gekennzeichnet, daß** der Linker die Struktur -CH₂-CH₂-SO₂-CH₂-CH₂- aufweist.

9. Konjugat nach einem der Ansprüche 1 bis 8, **dadurch gekennzeichnet, daß** zwischen X und S ein Spacer vorliegt.

10. Konjugat nach Anspruch 9, **dadurch gekennzeichnet, daß** der Spacer ein aliphatischer Rest ist.

11. Konjugat nach einem der Ansprüche 1 bis 10, **dadurch gekennzeichnet, daß** mehrere Liganden an den Träger gebunden sind.

12. Konjugat nach einem der Ansprüche 1 bis 11, **dadurch gekennzeichnet, daß** der Träger eine Chromatographiematrix, eine Mikrotiterplatte, eine Membran, eine Hohlfaser, Glas, ein magnetisches Teilchen, ein unlösliches oder lösliches Makromolekül, ein Lipid, eine Ladung-tragende chemische Gruppe, ein Photolabel, Biotin, Avidin, ein Peptid, ein Protein, ein Antigen, ein Arzneimittel, ein Enzym, ein strahlendichtendes Partikel, ein Immunglobulin und/oder eine Nukleinsäure ist.

13. Verfahren zur Herstellung eines Konjugats nach einem der Ansprüche 1 bis 12, **dadurch gekennzeichnet, daß** eine Verbindung X-SH, wobei zwischen X und S ggf. ein Spacer vorliegt, kovalent an einen Träger oder ggf. über einen Linker an den Träger gebunden wird.

14. Verwendung eines Konjugats nach einem der Ansprüche 1 bis 12 zur Bindung von Substanzen.

15. Verwendung nach Anspruch 14, wobei die Substanz ein Protein ist.

16. Verwendung nach Anspruch 15, wobei das Protein ein Immunglobulin ist.

## Claims

1. A conjugate comprising a ligand and a carrier, wherein the ligand has the general structure (1)
X-S- (1)
wherein
X is a quinazoline or derivative thereof, and
S is a sulfur atom.

2. The conjugate according to claim 1, **characterized in that** the quinazoline derivative is a quinazolinone or quinazolindione.

3. The conjugate according to claim 2, **characterized in that** the quinazolinone is a 4-quinazolinone.

4. The conjugate according to claim 2, **characterized in that** the quinazolindione is a 2,4-quinazolindione.

5. The conjugate according to any of claims 1 to 4, **characterized in that** X is substituted with an aliphatic, aromatic and/or heterocyclic residue.

6. The conjugate according to any of claims 1 to 5, **characterized in that** the quinazoline or derivative thereof is bound via position 2 or 3 of the quinazoline ring.

7. The conjugate according to any of claims 1 to 6, **characterized in that** a linker is present between S and the carrier.

8. The conjugate according to claim 7, **characterized in that** the linker has the structure -CH₂-CH₂-SO₂-H₂-CH₂-.

9. The conjugate according to any of claims 1 to 8, **characterized in that** a spacer is present between X and S.

10. The conjugate according to claim 9, **characterized in that** the spacer is an aliphatic residue.

11. The conjugate according to any of claims 1 to 10, **characterized in that** several ligands are bound to the carrier.

12. The conjugate according to any of claims 1 to 11, **characterized in that** the carrier is a chromatography matrix, microtitration plate, membrane, hollow fiber, glass, magnetic particle, insoluble or soluble macromolecule, lipid, charge-bearing chemical group, photolabel, biotin, avidin, peptide, protein, antigen, medicament, enzyme, radiation-sealing particle, immunoglobulin and/or nucleic acid.

13. The method of producing a conjugate according to any of claims 1 to 12, **characterized in that**, while a spacer is optionally present between X and S, a compound X-SH is bonded covalently to a carrier or is optionally bonded via a linker to the carrier.

14. Use of a conjugate according to any of claims 1 to 12 for binding substances.

15. Use according to claim 14, wherein the substance is a protein.

16. Use according to claim 15, wherein the protein is an immunoglobulin.

## Revendications

1. Conjugué comportant un ligand et un support, où le ligand présente la structure générale (1) :
X-S- (1)
dans laquelle
X est une quinazoléine ou un dérivé de celle-ci
S est un atome de soufre.

2. Conjugué selon la revendication 1, **caractérisé en ce que** le dérivé de quinazoléine est une quinazoléinone ou une quinazoléinedione.

3. Conjugué selon la revendication 2, **caractérisé en ce que** la quinazoléinone est une 4-quinazoléinone.

4. Conjugué selon la revendication 2, **caractérisé en ce que** la quinazoléinedione est une 2,4- quinazoléinedione.

5. Conjugué selon l'une quelconque des revendications 1 à 4, **caractérisé en ce que** X est substitué par un résidu aliphatique, aromatique et/ou hétérocyclique.

6. Conjugué selon l'une quelconque des revendications 1 à 5, **caractérisé en ce que** la quinazoléine ou son dérivé est relié par la position 2 ou 3 du cycle de quinazoléine.

7. Conjugué selon l'une quelconque des revendications 1 à 6, **caractérisé en ce qu'**entre S et le support est prévue une liaison.

8. Conjugué selon la revendication 7, **caractérisé en ce que** la liaison présente la structure -CH₂-CH₂-SO₂-CH₂-CH₂-.

9. Conjugué selon l'une quelconque des revendications 1 à 8, **caractérisé en ce qu'**entre X et S est prévu un espacement.

10. Conjugué selon la revendication 8, **caractérisé en ce que** l'espacement est un résidu aliphatique.

11. Conjugué selon l'une quelconque des revendications 1 à 10, **caractérisé en ce que** plusieurs ligands sont liés au support.

12. Conjugué selon l'une quelconque des revendications 1 à 11, **caractérisé en ce que** le support est une matrice pour chromatographie, une plaque de microtitration, une membrane, une fibre creuse, du verre, une particule magnétique, une macromolécule insoluble ou soluble, un lipide, un groupe chimique partant une charge, un photomarqueur, de la biotine, de l'avidine, un peptide, une protéine, un antigène, un médicament, une enzyme, une particule imperméable aux rayons, une immunoglobuline et/ou un acide nucléique.

13. Procédé pour la production d'un conjugué selon l'une quelconque des revendications 1 à 12 **caractérisé en ce qu'**un composé X-SH, où entre X et S est le cas échéant prévu un espacement, est lié de manière covalente à un support ou, le cas échéant, par une liaison au support.

14. Utilisation d'un conjugué selon l'une quelconque des revendications 1 à 12 pour la liaison de substances.

15. Utilisation selon la revendication 14, où la substance est une protéine.

16. Utilisation selon la revendication 15 où la protéine est une immunoglobuline.
